# EUROPÄISCHE PATENTSCHRIFT

(11) **EP 2 429 377 B1**
(45) Veröffentlichungstag und Bekanntmachung des Hinweises auf die Patenterteilung: **19.03.2014**
(21) Anmeldenummer: 10720748.2
(22) Anmeldetag: 14.05.2010
(51) Int. Cl.: A61B 1/07, A61B 17/42

(54) **MEDIZINISCHES INSTRUMENT**
MEDICAL INSTRUMENT
INSTRUMENT MÉDICAL

(30) Priorität: 15.05.2009 AT 7642009
(43) Veröffentlichungstag der Anmeldung: 21.03.2012
(73) Patentinhaber: Klaffenböck, Johann, 5350 Strobl (AT); Klaffenböck, Lukas, 5350 Strobl (AT)
(72) Erfinder: Klaffenböck, Johann, 5350 Strobl (AT); Klaffenböck, Lukas, 5350 Strobl (AT)
(74) Vertreter: Babeluk, Michael
(86) Internationale Anmeldenummer: PCT/EP2010/056656
(87) Internationale Veröffentlichungsnummer: WO 2010/130829

(56) Entgegenhaltungen:
- DE-A1- 4 435 644
- DE-A1-102006 052 086
- US-A- 5 361 583

## Beschreibung

Die Erfindung betrifft ein medizinisches Instrument mit einem länglichen Element, das vorzugsweise für die transorale Platzierung in einem Magen konfiguriert ist, und einem an einem Ende des länglichen Elements anordenbaren Endeffektor, wobei der Endeffektor über einen hydraulischen Betätigungsmechanismus betätigbar ist, sowie ein Verfahren zum Betätigen eines Endeffektors, der insbesondere an einem Ende eines Endoskopiesystems angeordnet ist.

In der DE 44 35 644 B1 wird ein hydraulischer Biegemechanismus für einen Biegeabschnitt eines Endoskopiesystems beschrieben. Eine ähnliche Vorrichtung kann auch der EP 0 401 129 A1 entnommen werden.

In der US 4,485,817 A ist ein hydraulischer Betätigungsmechanismus für einen Endeffektor mit den Merkmalen des Oberbegriffs des Anspruchs 1 beschrieben, bei welchem ein Kolben gegen einen zweiten bewegt wird, um den für die Betätigung des Endeffektors benötigten Druck in der Hydraulikflüssigkeit aufzubauen. Nachteilig an diesem hydraulischen Betätigungsmechanismus ist insbesondere dessen komplizierter Aufbau sowie der Tatsache, dass dieser Betätigungsmechanismus die Ansteuerung von lediglich zwei definierten Betriebszustände des Endeffektors erlaubt. Insbesondere bei der Magenplikation sind jedoch häufig mehr als zwei Betriebszustände des Endeffektors notwendig, sodass der Betätigungsmechanismus für derartige Endeffektoren ungeeignet ist. Eine ähnliche Vorrichtung ist auch in der US 5,361,583 A gezeigt.

Es ist daher Aufgabe der Erfindung, ein medizinisches Instrument mit einem hydraulischen Betätigungsmechanismus bereitzustellen, der einfach im Aufbau und damit sicher im Betrieb ist und die Nachteile des Stand der Technik beseitigt.

Diese Aufgabe wird erfindungsgemäß durch ein medizinisches Instrument der eingangs erwähnten Art dadurch gelöst, dass der hydraulische Betätigungsmechanismus einen Zylinder mit zumindest einem, vorzugsweise zwei, drei, vier oder fünf Überströmkanälen für eine Hydraulikflüssigkeit, die über zumindest eine Bohrung mit dem Zylinderinneren verbunden sind, aufweist, ein in dem Zylinder angeordneter, entlang der Längsachse des Zylinders bewegbarer Kolben vorgesehen ist, wobei der Kolben den Innenraum des Zylinders in einen ersten Zylinderraum und in einen zweiten Zylinderraum teilt und über eine Kolbenstange mit dem Endeffektor in Verbindung steht, und zumindest ein Anschluss zur Einleitung von Hydraulikflüssigkeit in den ersten Zylinderraum sowie zumindest ein weiterer Anschluss, der mit zumindest einem Überströmkanal des Zylinders in Verbindung steht, vorgesehen sind.

Die Überströmkanäle sind hierbei in einer ersten Ausführung der Erfindung als Vertiefungen an der Außenfläche der Zylinderwand ausgebildet und sind über Bohrungen mit dem Innenraum des Zylinders verbunden, wobei die Bohrungen einen unterschiedlichen Abstand zu einem Ende des Zylinders aufweisen. Der Zylinder ist bevorzugt in einer Hülse einsetzbar, sodass die Überströmkanäle zwischen Hülseninnenwand und Zylinderaußenwand durch die Vertiefungen in der Zylinderaußenwand gebildet werden.

Bevorzugterweise sind die mit dem Innenraum des Zylinders in Verbindung stehenden Anschlüsse für die Hydraulikflüssigkeit in einem Dichtelement angeordnet, das an dem der Kolbenstange abgewandten Ende der Hülse angeordnet ist, um einen kompakten und betriebssicheren hydraulischen Betätigungsmechanismus zu erhalten.

In einer alternativen Ausführung der Erfindung ist der zumindest eine Überströmkanal innerhalb der Zylinderwand angeordnet und steht über zumindest eine Bohrung mit dem Zylinderraum in Verbindung. Zwar ist diese Ausführung der Erfindung teurer in der Herstellung als die vorgenannte, allerdings wird hier kein eigenes Dichtelement mehr benötigt, weil die Anschlüsse für die Hydraulikflüssigkeit direkt im Zylinderboden untergebracht sind. Damit lässt sich die Baugröße weiter reduzieren, was insbesondere im endoskopischen Anwendungsbereich wichtig ist.

Wird nun über jenen Anschluss, der mit dem benachbarten ersten Zylinderraum in Verbindung steht, Hydraulikflüssigkeit eingepumpt, bewegt sich der Kolben von dem Zylinderboden weg, während über einen zweiten Anschluss, der über einen Überströmkanal mit dem zweiten Zylinderraum in Verbindung steht, Hydraulikflüssigkeit aus dem zweiten Zylinderraum strömt. Damit vergrößert sich der erste Zylinderraum, während der zweite Zylinderraum sich verkleinert, und die Kolbenstange des Kolbens wird aus dem Zylinder herausgedrückt. Sobald die zumindest eine Bohrung des Überströmkanals von dem in dem Zylinder befindlichen Kolbenhemd des Kolbens verschlossen wird, kann sich der Kolben aufgrund des Gegendrucks innerhalb des zweiten Zylinderraums nicht mehr weiterbewegen und eine definierte Position des Kolbens ist erreicht. Diese Position des Kolbens entspricht einem definierten Betriebszustand des mit der Kolbenstange in Verbindung stehenden Endeffektors. Je nach Anzahl und Position der Überströmkanäle bzw. Bohrungen der Überströmkanäle können unterschiedliche Betriebszustände des an das erfindungsgemäße medizinische Instrument anschließbaren Endeffektors definiert werden.

So sind beispielsweise für die Betätigung eines Endeffektors mit zwei Greifelementen zur Verwendung in der Magenplikation drei Betriebszustände notwendig: eine erste Schließposition, bei welcher der Endeffektor beispielsweise in dem Magen eines Patienten platziert wird; eine Offenposition, aus welcher durch Bewegung der Greifelemente zueinander Gewebe zwischen den Greifelementen angeordnet wird und schließlich eine zweite Schließposition, bei welcher eine Klammereinrichtung das zwischen den Greifelementen des Endeffektors befindliche Gewebe vernäht. Ein hierfür einsetzbarer Endeffektor kann beispielsweise der EP 1 187 559 A1 oder der US 2004/59349 A1 entnommen werden.

Daher weist in einer bevorzugten Ausführung der Erfindung, die besonders für derartige Endeffektoren für die Magenplikation geeignet ist, vier Überströmkanäle auf, wobei drei hiervon jeweils die obenbeschriebenen Betriebszustände des Endeffektors bewirken, während der vierte Überströmkanal dem vollständigen Druckablass aus dem zweiten Zylinderraum dient.

Selbstverständlich können auch andere Endeffektoren an das erfindungsgemäße medizinische Instrument angeschlossen werden, wobei hierfür bevorzugterweise ein standardisiertes Adapterelement an dem medizinischen Instrument vorgesehen ist. Als Endeffektoren können unterschiedlichste Greifelemente, aber auch Sonden und andere endoskopische und/oder endochirurgische Elemente an das erfindungsgemäße Hydrauliksystem angeschlossen werden.

Zum Betreiben des erfindungsgemäßen hydraulischen Betätigungsmechanismus ist bevorzugterweise eine Hydraulikeinrichtung, die mit dem hydraulischen Betätigungsmechanismus zusammenwirkt, vorgesehen, wobei die Hydraulikeinrichtung zumindest eine Pumpe sowie zumindest je eine Hydraulikleitung zu jedem Anschluss des Dichtelements aufweist, und vorzugsweise jeder Hydraulikleitung zumindest ein Schaltelement zugeordnet ist.

Des weiteren wird die Aufgabe durch ein endochirurgisches Instrument, insbesondere ein Endoskop, mit einem länglichen Element, das vorzugsweise für die transorale Platzierung in einem Magen konfiguriert ist, und ein an einem Ende des länglichen Elements angeordneten Endeffektor aufweist, gelöst, wobei der Endeffektor über die erfindungsgemäße Vorrichtung hydraulisch betätigbar ist.

Hierbei weist der Endeffektor bevorzugterweise zwei klauenartige Greifelemente auf, die aus einer Offenstellung in zumindest eine Schließstellung, vorzugsweise zwei Schließstellungen oder auch mehr zueinander bewegbar sind.

In einem Verfahren zum Betätigen eines Endeffektors, der insbesondere an einem Ende eines endochirurgischen Instruments angeordnet ist, wird unter Verwendung der erfindungsgemäßen Vorrichtung die Position eines Kolben mit einer Kolbenstange innerhalb eines Zylinders durch Einströmen von Hydraulikflüssigkeit in einen ersten Zylinderraum und/oder durch Ausströmen von Hydraulikflüssigkeit aus einen zweiten Zylinderraum des Zylinders verändert, wobei die Kolbenposition den Betriebszustand des mit der Kolbenstange in Verbindung stehenden Endeffektors bestimmt.

Aufgrund der extrem platzsparenden Bauweise des erfindungsgemäßen medizinischen Instruments kann es auch in Verbindung mit bekannten endoskopischen System eingesetzt werden. So kann der Durchmesser des erfindungsgemäßen medizinischen Instruments derart gering gehalten werden, dass es beispielsweise in einem Kanal eines Endoskops gemäß dem Stand der Technik angeordnet werden kann.

Im folgenden wird anhand von nicht einschränkenden Ausführungsbeispielen mit zugehörigen Figuren die Erfindung näher erläutert. Darin zeigen:
- Fig. 1: eine explodierte Darstellung des erfindungsgemäßen hydraulischen Betätigungsmechanismus;
- Fig. 2a: eine erste Schnittdarstellung des Betätigungsmechanismus aus Fig. 1 im zusammengebauten Zustand;
- Fig. 2b: eine zweite, um 90° zu der Schnittdarstellung aus Fig. 2a gedrehte Schnittdarstellung des Betätigungsmechanismus;
- Fig. 2c: eine Ansicht des Betätigungsmechanismus von der Seite des Dichtelements betrachtet;
- Fig. 2d: eine Ansicht des Betätigungsmechanismus von der Seite der Kolbenstange betrachtet;
- Fig. 3a: eine Schnittansicht des Betätigungsmechanismus mit Endeffektor;
- Fig. 3b: eine Seitenansicht des Betätigungsmechanismus mit Endeffektor aus Fig. 3a;
- Fig. 3c: eine perspektivische Ansicht des Betätigungsmechanismus mit Endeffektor aus Fig. 3a;
- Fig. 4a: ein Hydraulikschema für den geöffneten Endeffektor gemäß Fig. 3a bis Fig. 3c;
- Fig. 4b: der geöffnete Endeffektor aus Fig. 3a mit Abdeckung in einer perspektivischen Ansicht;
- Fig. 5a: ein Hydraulikschema für den Endeffektor in einem ersten Schließzustand;
- Fig. 5b: eine perspektivischen Ansicht der Endeffektor in einem ersten Schließzustand;
- Fig. 5c: eine Ansicht des Endeffektors aus Fig. 5b von oben;
- Fig. 6a: ein Hydraulikschema für den Endeffektor in einem zweiten Schließzustand;
- Fig. 6b: eine perspektivischen Ansicht des Endeffektors in einem zweiten Schließzustand;
- Fig. 6c: eine Ansicht des Endeffektors aus Fig. 6b von oben;
- Fig. 6d: ein Hydraulikschema zum Öffnen des Endeffektors aus dem zweiten Schließzustand;
- Fig. 7a: eine teilweise geschnittene Ansicht eines weiteren Endeffektors;
- Fig. 7b: eine perspektivische Ansicht des Endeffektors aus Fig. 7a;
- Fig. 7c: ein Hydraulikschema für den Endeffektor aus Fig. 7a; und
- Fig. 8a, Fig. 8b: zwei teilweise geschnittene Ansichten eines Rückstellelementes.

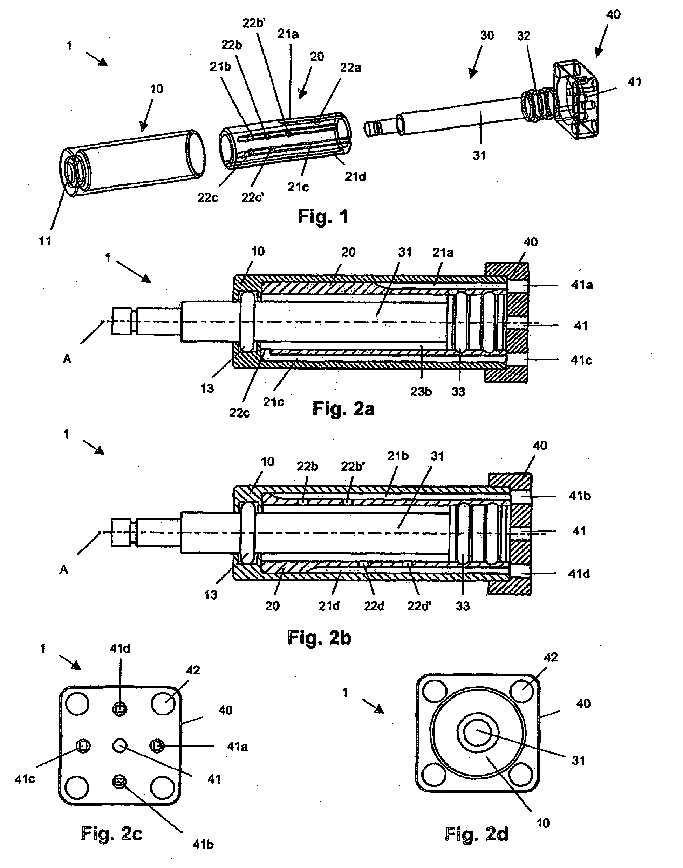

In der Fig. 1 ist in einer explodierten Darstellung der erfindungsgemäße hydraulische Betätigungsmechanismus 1 insbesondere für ein endochirurgisches und/ oder endoskopisches Instrument dargestellt. Er weist eine Hülse 10 auf, in die ein Zylinder 20 einsteckbar ist. Der Zylinder 20 weist in der dargestellten Ausführung vier Überströmkanäle 21a, 21b, 21c, 21d auf, die als parallel zur Längsachse A des Zylinders 20 angeordnete Einfräsungen am Außenmantel des Zylinders 20 ausgebildet sind und mit dem Inneren 23 (Figs. 2a, 2b) des Zylinders 20 über Bohrungen 22a, 22b, 22b', 22c, 22c', 22d, 22d' in Verbindung stehen. Des weiteren weist der hydraulische Betätigungsmechanismus 1 einen Kolben 30 mit einer Kolbenstange 31 und einem dichtenden Kolbenhemd 32 auf, der in dem Zylinder 20 entlang dessen Längsachse A bewegbar ist. Schließlich ist ein Dichtelement 40 vorgesehen, das über fünf, als Bohrungen ausgebildete Anschlüsse 41, 41a, 41b, 41c, 41d für die Leitung von Hydraulikflüssigkeit durch das Dichtelement 40 verfügt.

In den Fig. 2a und Fig. 2b ist der hydraulische Betätigungsmechanismus 1 im zusammengesetzten Zustand dargestellt. Hierbei ist der Zylinder 20 in der Hülse 10 angeordnet und mittels Dichtelement 40 in seiner Position fixiert. Die Anordnung von Hülse 10, Zylinder 20 und Dichtelement 40 wird derart vorgenommen, dass die Hydraulikanschlüsse 41a, 41b, 41c, 41d des Dichtelements 40 in fluchtender Verbindung mit den Überströmkanälen 21a, 21b, 21c, 21d des Zylinders 20 angeordnet sind, wobei die Überströmkanäle 21a, 21b, 21c, 21d über Bohrungen 22a, 22b, 22b', 22c, 22c', 22d, 22d' mit dem Innenraum des Zylinders 20 in Verbindung stehen.

Des weiteren ist der Kolben 30 im Inneren des Zylinders 20 angeordnet, wobei dessen Kolbenstange 31 durch eine zentrale Öffnung 11 der Hülse 10 geführt ist. Der Kolben 30 weist an seinem innerhalb des Zylinders 20 angeordneten Kolbenhemd 32 Dichtmittel 33 auf, die das Kolbenhemd 32 ringförmig umgeben und in entsprechende Stellung des Kolbens 30 innerhalb des Innenraums 23 des Zylinders 20 zumindest einen Überströmkanal 21a, 21b, 21c, 21d verschließen (Fig. 2a). Ein weiteres Dichtmittel 13 ist in der konzentrischen Öffnung 11 der Hülse 10 vorgesehen, um ein Auslaufen von Hydraulikflüssigkeit aus dem Zylinder 20 zu verhindern.

In der Fig. 2c ist eine Draufsicht auf den hydraulischen Betätigungsmechanismus 1 gezeigt, bei welcher im Wesentlichen das Dichtelement 40 sichtbar ist. Hierbei ist erkennbar, dass um einen zentralen Hydraulikanschluss 41 vier weitere Anschlüsse 41a, 41b, 41c, 41d auf der im Wesentlichen quadratischen Dichtplatte 40 angeordnet sind. In den Ecken der Dichtplatte 40 sind des weiteren Ausnehmungen 42, die der Aufnahme von Fixierelementen (nicht dargestellt) zur Befestigung der Dichtplatte 40 an der Hülse 10 dienen, vorgesehen. In der Fig. 2d ist schließlich eine Draufsicht auf den erfindungsgemäßen hydraulischen Betätigungsmechanismus 1 gezeigt, wobei hier die Betrachtung auf die Kolbenstange 31 erfolgt.

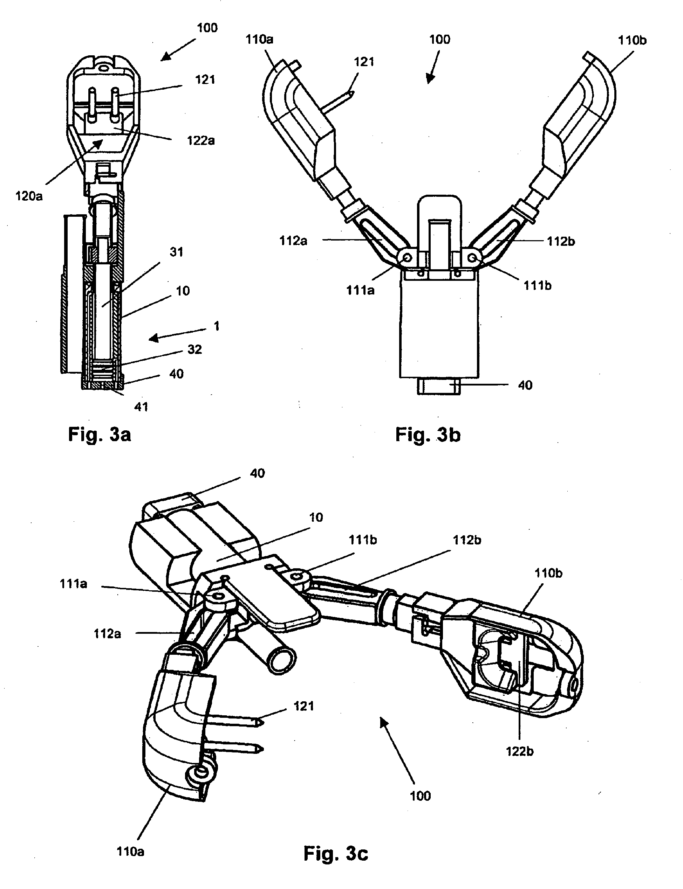

In den Fig. 3a bis Fig. 3c ist der Betätigungsmechanismus 1 mit daran befestigtem Endeffektor 100, wie er insbesondere bei endochirurgischen Eingriffen zum Einsatz kommt, dargestellt. In der Fig. 3a ist gezeigt, dass der Endeffektor 100 an der Kolbenstange 31 des Kolbens 30 angeordnet ist, wobei sich der Endeffektor 100 wie in der Fig. 3b gezeigt in einer Offenstellung befindet. Der Endeffektor 100 weist zwei klauenartige Greifelemente 110a, 110b auf, wobei in dem einen Greifelement 110a ein erster Teil 120a eines Klammerelements angeordnet ist, das über zwei Stifte 121, die auf einem Plattenelement 122a angeordnet sind, verfügt. Eine derartige Klammereinrichtung kann beispielsweise der österreichischen Patentanmeldung A 239/2009 des Anmelders entnommen werden.

Durch Schließen der beiden Greifelemente 110a, 110b des Endeffektors 100 mit Hilfe des erfindungsgemäßen hydraulischen Betätigungsmechanismus 1 wird der erste Teil 120a der Klammereinrichtung mit einem im zweiten Greifelement 110b angeordneten zweiten Teil 122b der Klammereinrichtung in Eingriff gebracht, um beispielsweise Gewebe im Zuge einer Magenplikation zu fixieren. Um die beiden Greifelemente 110a, 110b zueinander hin- und voneinander wegbewegen zu können, sind Führungselemente 111a, 111b vorgesehen, die bei Bewegung des Kolbens 30 des hydraulischen Betätigungsmechanismus 1 entlang von Führungsnuten 112a, 112b bewegt werden. In der Fig. 3a ist der Kolben 30 an seiner untersten Position, bei welcher er im Wesentlichen an die Dichtplatte 40 anliegt, dargestellt. Wird der Kolben 30 mit Hilfe von Hydraulikflüssigkeit, die über die zentrale Bohrung 41 des Dichtelements 40 einströmt, nach oben bewegt, so werden die Führungselemente 111a, 111b entlang der Führungsnuten 112a in Richtung der Greifelemente 110a, 110b bewegt, so dass sich in Folge diese aufgrund der Lage der Führungsnuten 112a, 112b aufeinanderzubewegen. Die gekrümmte Lage der Führungsnuten 112a, 112b hat den Vorteil, dass bei einem ersten Schließen des Endeffektors 100 eine geringe Kraft benötigt wird, während in der zweiten Schließposition die für das Abbrechen der Stifte 121 benötigte höhere Kraft aufgrund der speziellen Lage der Führungsnuten 112a, 112b geliefert wird. Die Führungsnuten 112a, 112b können hierbei je nach Anwendungsfall gerade oder gekrümmt verlaufend ausgeführt sein.

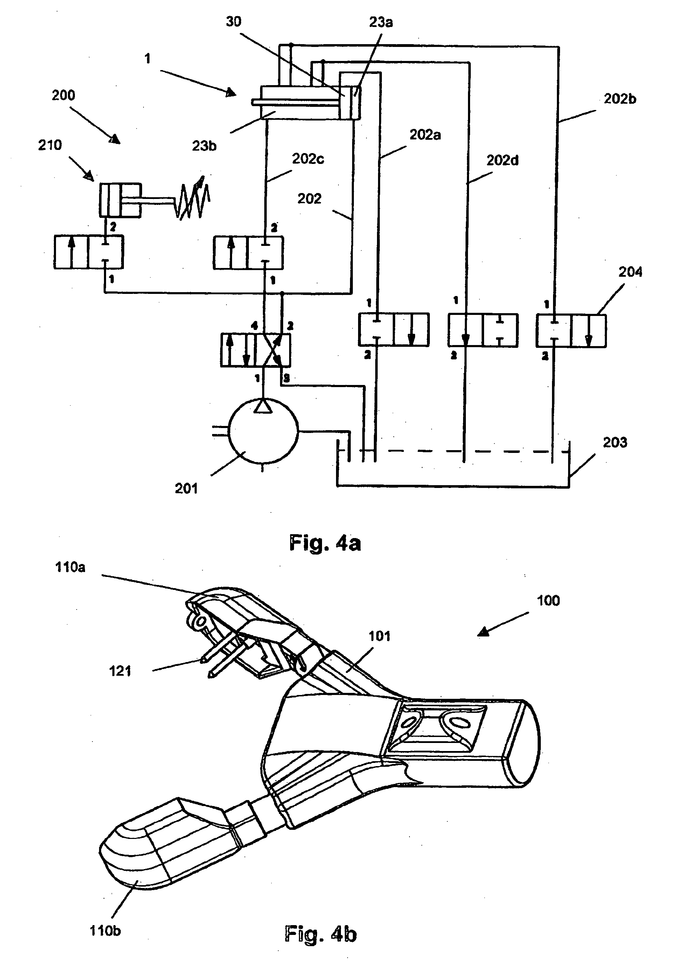

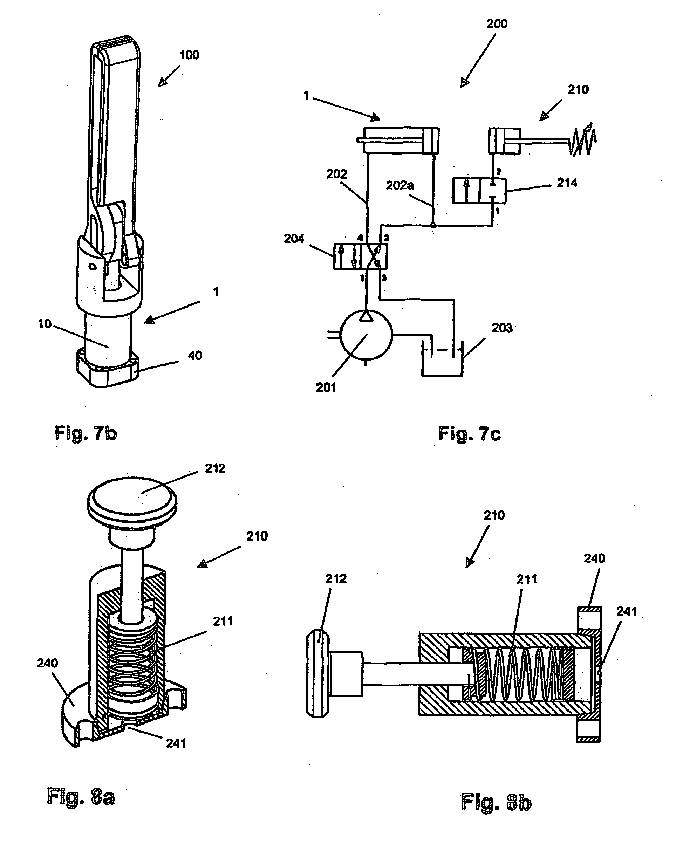

Im folgenden wird die Funktionsweise des erfindungsgemäßen hydraulischen Betätigungsmechanismus 1 näher erläutert. Hierfür ist in den Fig. 4a bis Fig. 6d eine für den Betrieb des Betätigungsmechanismus 1 geeignete Hydraulikeinrichtung 200 schematisch dargestellt. Die Hydraulikeinrichtung 200 weist eine Pumpe 201 auf, mit deren Hilfe Hydraulikflüssigkeit zu dem hydraulischen Betätigungsmechanismus 1 über eine erste Hydraulikleitung 202, die mit einem ersten Zylinderraum 23a in Verbindung steht, zugeführt wird. Des weiteren sind die Überströmkanäle 21a, 21b, 21c, 21d an weitere Hydraulikleitungen 202a, 202b, 202c, 202d angeschlossen und stehen mit einem Reservoir 203 für Hydraulikflüssigkeit in Verbindung. Jede Hydraulikleitung 202, 202a, 202b, 202c, 202d ist mit zumindest einem Schaltelement 204, beispielsweise einem Hydraulikventil, ausgestattet, das ein Schalten und damit ein Fließen der Hydraulikflüssigkeit in der jeweiligen Hydraulikleitung 202, 202a, 202b, 202c, 202d erlaubt. In manchen Fällen ist für die korrekte Betätigung des Endeffektors 100 ein zusätzliches Rückstellelement 210 von Vorteil, das in den Figuren 8a und 8b im Detail beschrieben ist.

In der Fig. 4a ist jene Schaltung dargestellt, bei welcher der Kolben 30 des hydraulischen Betätigungsmechanismus 1 in seiner untersten Position in unmittelbarere Nachbarschaft zur Dichtplatte 40 gezeigt ist. Zur klaren Darstellung wurde hier auf die Darstellung der Dichtplatte 40 mit davon abzweigenden Hydraulikleitungen verzichtet. Die Hydraulikleitungen 202, 202a, 202b, 202c, 202d sind direkt in einer Position an den Überströmkanäle 21, 21a, 21b dargestellt, während in der Ausführung der Erfindung gemäß den Figs. 1 bis Fig. 2d die Hydraulikleitungen 202, 202a, 202b, 202c, 202d tatsächlich an dem Dichtelement 40 angeschlossen sind. Das Hydraulikschema gemäß Fig. 4a zeigt jenen Zustand der Hydraulikeinrichtung 200, bei welcher der Endeffektor 100 geöffnet ist, wie er in den Fig. 3a bis Fig. 3c bzw. Fig. 4b gezeigt ist. In der Fig. 4b ist der Endeffektor 100 gemäß der Fig. 3a mit der dazugehörigen Abdeckung 101, beispielsweise aus Silikon, dargestellt.

Der hydraulische Betätigungsmechanismus 1 ist insbesondere auch für die Betätigung von Endeffektoren geeignet, die in der endoskopische Magenplikation eingesetzt werden. Bei der endoskopischen Magenplikation wird mit Hilfe eines Endoskops ein Endeffektor 100 in den Magen eingeführt und dieser anschließend derart betätigt, dass eine im Endeffektor 100 angeordnete Klammereinrichtung (wie beispielsweise jene in den Fig. 3a bis Fig. 3c dargestellt) im (Magen)Gewebe platziert wird. Beim Einführen des Endoskops in den Magen muss der Endeffektor 100 allerdings geschlossen sein, damit keine Verletzung der Speiseröhre oder des Magens beim Einführen erfolgt. Gleichzeitig jedoch darf der Endeffektor 100 nicht so weit geschlossen sein, dass die Stifte 121 der Klammereinrichtung in den zweiten plattenförmigen Teil 122b einrasten.

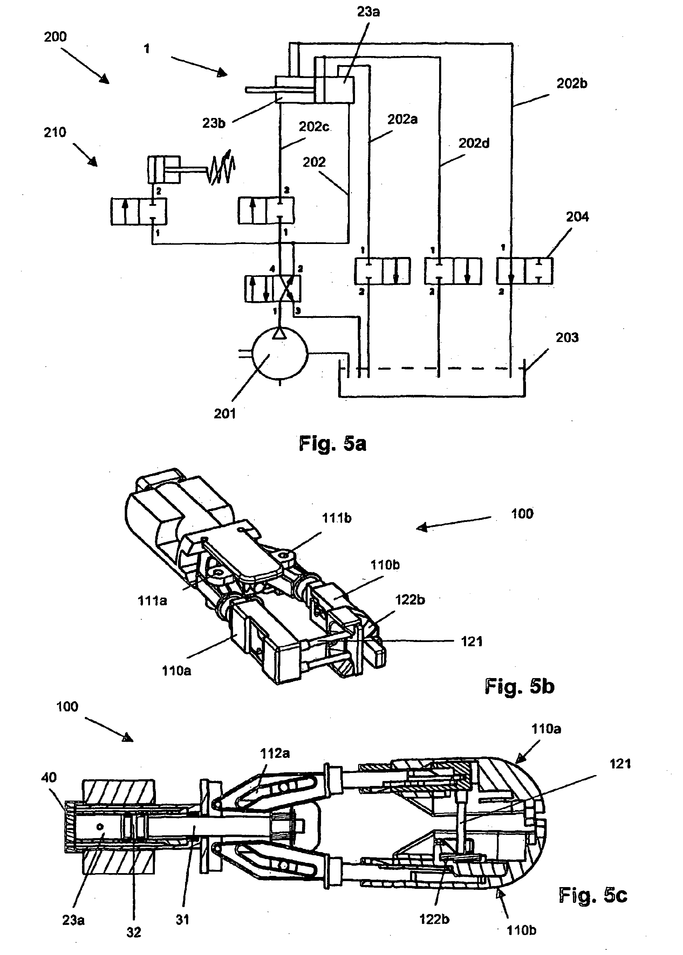

In den Fig. 5a bis Fig. 5c ist jene erste Schließstellung des Endeffektors 100, bei welchem die bevorzugt transorale Platzierung des Endeffektors 100 an die gewünschte Stelle erfolgt, mit zugehörigem Hydraulikschema dargestellt. Hierbei befindet sich der Kolben 30 innerhalb der Zylinders 20 in einer Mittelstellung und teilt den Innenraum des Zylinders in zwei Zylinderräume 23a und 23b. Sobald der Endeffektor 100 an der vorgesehenen Stelle, beispielsweise innerhalb des Magens platziert ist, werden die beiden Greifelemente 110a, 110b in die Offenposition gemäß Fig. 4b bewegt.

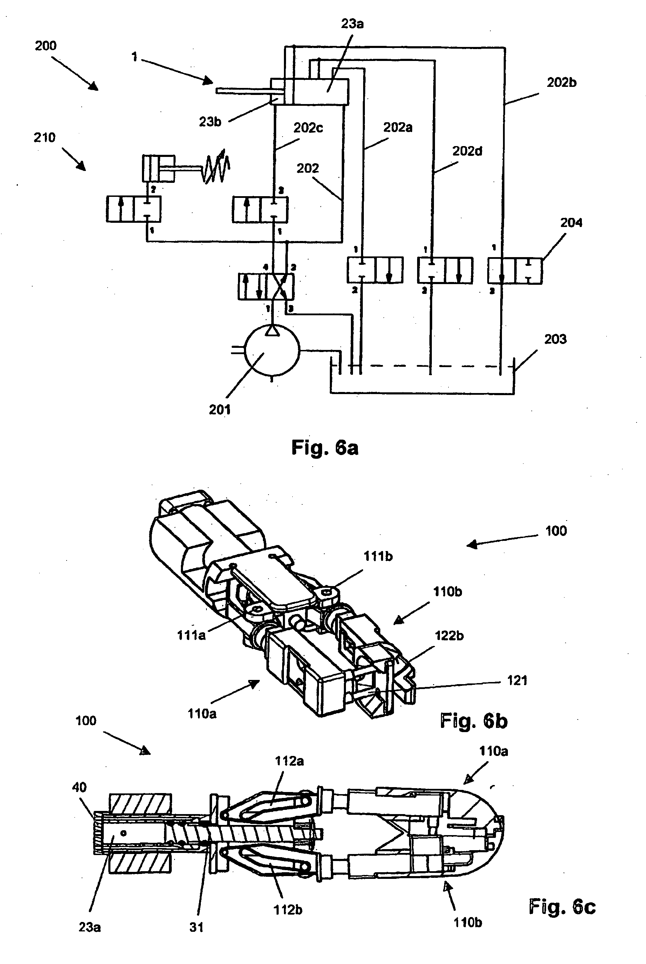

Durch anschließendes Schließen der Greifelemente 110a, 110b wird das Magengewebe von den Greifelementen 110a, 110b umfasst und von den Stiften 121 durchbohrt. Schließlich wird der Kolben 30 in eine oberste Position gemäß Fig. 6a bis 6c gebracht, bei welcher die Stifte 121 der Klammereinrichtung an der vorgesehenen Sollbruchstelle brechen (nicht dargestellt).

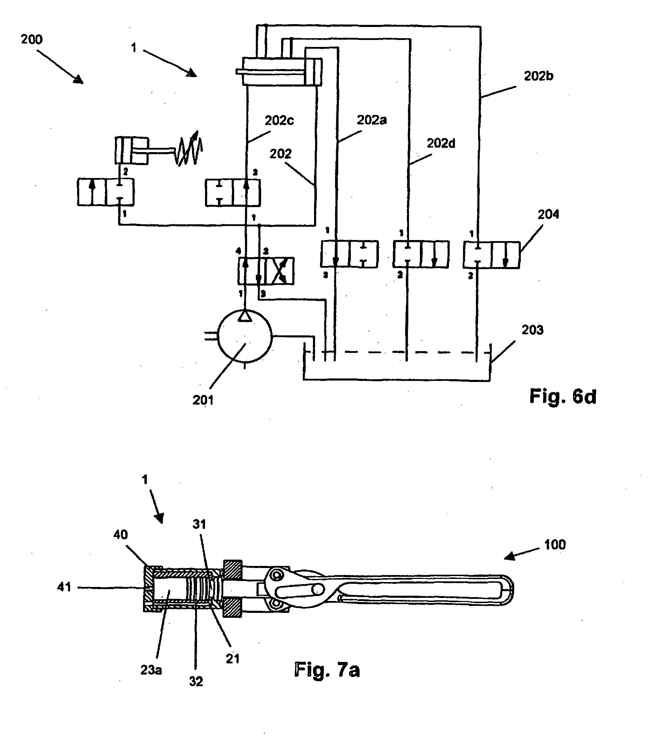

Sobald der Endeffektor 100 durch Bewegen des Kolbens 30 in die unterste Position in unmittelbarer Nachbarschaft zu dem Dichtelement 40 wieder geöffnet wird (Fig. 6d), wobei die Hydraulikflüssigkeit über den Überströmkanal 202d in den zweiten Zylinderraum 23b strömt, verbleibt die Klammereinrichtung im Gewebe und der Endeffektor 100 kann wiederum in der ersten Schließstellung gemäß den Fig. 5b und Fig. 5c aus dem Magen durch Herausziehen über die Speiseröhre sowie der Mundhöhle entfernt werden.

Selbstverständlich ist der erfindungsgemäße hydraulische Betätigungsmechanismus 1 nicht nur für den dargestellten Endeffektor 100 geeignet, sondern kann auch für andere insbesondere in der Medizin verwendete Endeffektoren, die üblicherweise über Bowdenzüge, Schub- oder Zugelemente betätigt werden, eingesetzt werden.

So können beispielsweise zangenartige Endeffektoren 100, wie in den Fig. 7a und Fig. 7b dargestellt, ebenfalls mit dem erfindungsgemäßen Betätigungsmechanismus 1 betätigt werden. Hierbei ist die Hydraulikeinheit 200, wie in Fig. 7c dargestellt, einfacher ausgeführt, weil hier lediglich zwei Positionen, nämlich eine Offenstellung sowie eine Geschlossenstellung des Endeffektors 100 angesteuert werden müssen. Daher weist der hydraulische Betätigungsmechanismus 1 in dieser Ausführung lediglich einen Überströmkanal 21 auf, während die Dichtplatte 40 über einen Hydraulikanschluss 41 für den Druckaufbau im Innenraum 23 des Zylinders 20 sowie einen Hydraulikanschluss 41a, verfügt, der mit dem Überströmkanal 21 in Verbindung steht. Selbstverständlich kann auch der zuvor beschriebene hydraulische Betätigungsmechanismus mit mehr als einem Überströmkanal zum Einsatz kommen.

In der geschlossenen Position des zangenartigen Endeffektors 100 gemäß Fig. 7a und 7b befindet sich der Kolben 30 in einer oberen Position, während zum Öffnen der Zange 100 der Kolben 30 durch Öffnen des Überströmkanals 21 in eine untere Position, bei welcher das Kolbenhemd 32 in unmittelbarer Nachbarschaft zur Dichtplatte 40 angeordnet ist, bewegt wird. Da diese Art von Endeffektoren 100 insbesondere zum Festhalten und/oder Fixieren von Gewebe über einen längeren Zeitraum während einer Operation eingesetzt werden, lässt die Haltekraft dieses Endeffektors insbesondere bei Betätigungsmechanismen mittels Bowdenzügen über einen längeren Zeitraum nach und kann im schlimmsten Fall zum Verlieren des festgehaltenen Gewebes führen. Daher ist bevorzugt ein zusätzliches Rückstellelement 210 vorgesehen, das den für die korrekte Funktion des Endeffektors 100 benötigten Hydraulikdruck federnd aufrecht erhält.

In den Fig. 8a und Fig. 8b wird eine bevorzugte Ausführung eines derartigen zusätzlichen Rückstellelementes 210 gezeigt. Das Rückstellelement 210 verfügt hierbei über eine Federeinrichtung 211, hier eine Feder, sowie über einen Drehknopf 212, mit dessen Hilfe die gewünschte Federkraft eingestellt werden kann. Über eine Dichtplatte 240 mit einem Hydraulikanschluss 241 steht das Rückstellelement 210 mit dem Hydrauliksystem in Verbindung und kann erforderlichenfalls über das Schaltelement 214 (Fig. 7c) aktiviert werden. Es versteht sich, dass dieses Rückstellelement auch in Verbindung mit anderen Endeffektoren aller Art zum Einsatz kommen kann.

Gegebenenfalls kann zusätzlich oder alternativ auch eine Signaleinrichtung vorgesehen sein, die einen Druckabfall innerhalb des Hydrauliksystems 200 anzeigt, wonach das Rückstellelement 210 vom Benutzer aktiviert werden kann.

Es versteht sich, dass die Erfindung nicht auf die dargestellten Ausführungsbeispiele beschränkt ist. So sind insbesondere die Anzahl und Position der Überströmkanäle an die jeweiligen Erfordernisse anpassbar, es können auch mehr als fünf Überströmkanäle vorgesehen sein.

## Patentansprüche

1. Medizinisches Instrument mit einem länglichen Element und einem an einem Ende des länglichen Elements anordenbaren Endeffektor (100), wobei der Endeffektor (100) über einen hydraulischen Betätigungsmechanismus (1) betätigbar ist, **dadurch gekennzeichnet, dass** der hydraulische Betätigungsmechanismus (1) einen Zylinder (20) mit zumindest einem, vorzugsweise zwei, drei, vier oder fünf Überströmkanälen (21a, 21b, 21c, 21d) für eine Hydraulikflüssigkeit, die über zumindest eine Bohrung (22a, 22b, 22b', 22c, 22c', 22d, 22d') mit dem Zylinderinneren verbunden sind, aufweist, ein in dem Zylinder (20) angeordneter, entlang der Längsachse des Zylinders (20) bewegbarer Kolben (30) vorgesehen ist, wobei der Kolben (30) den Innenraum (23) des Zylinders (20) in einen ersten Zylinderraum (23a) und in einen zweiten Zylinderraum (23b) teilt und über eine Kolbenstange (31) mit dem Endeffektor (100) in Verbindung steht, und zumindest ein Anschluss (41) zur Einleitung von Hydraulikflüssigkeit in den ersten Zylinderraum (23a) sowie zumindest ein weiterer Anschluss (41a, 41b, 41c, 41d), der mit zumindest einem Überströmkanal (21a, 21b, 21c, 21d) des Zylinders (20) in Verbindung steht, vorgesehen sind.

2. Medizinisches Instrument nach Anspruch 1, **dadurch gekennzeichnet, dass** die zumindest eine Bohrung (22a, 22b, 22b', 22c, 22c', 22d, 22d') zumindest eines Überströmkanals (21a, 21b, 21c, 21d) zwischen einem oberen und einem unteren Kolbenanschlag angeordnet ist, sodass der Kolben (30) innerhalb des Zylinders (20) in zumindest eine definierte Mittelstellung, die zwischen dem oberen und dem unteren Kolbenanschlag angeordnet ist, bringbar ist.

3. Medizinisches Instrument nach Anspruch 1 oder 2, **dadurch gekennzeichnet, dass** der zumindest eine Überströmkanal (21a, 21b, 21c, 21d) an der Außenfläche des Zylinders (20) als Vertiefung ausgebildet ist und über die zumindest eine Bohrung (22a, 22b, 22b', 22c, 22c', 22d, 22d') mit dem Zylinderraum (23a, 23b) in Verbindung steht, wobei der Zylinder (20) in eine Hülse (10) einsetzbar ist.

4. Medizinisches Instrument nach Anspruch 3, **dadurch gekennzeichnet, dass** die mit dem Innenraum (23) des Zylinders (20) in Verbindung stehenden Anschlüsse (41, 41a, 41b, 41c, 41d) für die Hydraulikflüssigkeit in einem Dichtelement (40) angeordnet sind, das an dem der Kolbenstange (31) abgewandten Ende der Hülse (10) angeordnet ist.

5. Medizinisches Instrument nach Anspruch 1 oder 2, **dadurch gekennzeichnet, dass** der zumindest eine Überströmkanal (21a, 21b, 21c, 21d) innerhalb der Zylinderwand angeordnet ist und über zumindest eine Bohrung (22a, 22b, 22b', 22c, 22c', 22d, 22d') mit dem Zylinderraum (23a, 23b) in Verbindung steht.

6. Medizinisches Instrument nach einem der Ansprüche 1 bis 5, **dadurch gekennzeichnet, dass** je Überströmkanal (21a, 21b, 21c, 21d) zwei Bohrungen (22a, 22b, 22b', 22c, 22c', 22d, 22d') vorgesehen sind.

7. Medizinisches Instrument nach einem der Ansprüche 1 bis 6, **dadurch gekennzeichnet, dass** der Endeffektor (100) zwei klauenartige Greifelemente (110a, 110b) aufweist, die aus einer Offenstellung in zumindest eine Schließstellung, vorzugsweise in zwei Schließstellungen zueinander bewegbar sind.

8. Medizinisches Instrument nach einem der Ansprüche 1 bis 7, **dadurch gekennzeichnet, dass** eine Vorrichtung zur Betätigung eines Endeffektors des medizinischen Instruments mit einer Hydraulikeinrichtung (200) vorgesehen ist, die mit dem hydraulischen Betätigungsmechanismus (1) des medizinischen Instruments zusammenwirkt, wobei die Hydraulikeinrichtung (200) zumindest eine Pumpe (201) sowie zumindest je eine Hydraulikleitung (202, 202a, 202b, 202c, 202d) zu jedem Anschluss (41, 41a, 41b, 41c, 41d) des Dichtelements (40) aufweist, und vorzugsweise jeder Hydraulikleitung (202, 202a, 202b, 202c, 202d) zumindest ein Schaltelement (204) zugeordnet ist.

## Claims

1. Medical instrument comprising an and an end effector (100) positioned at one end of the elongated element wherein the elongated element is operated by means of a hydraulic actuating mechanism (1), **characterised in that** said hydraulic actuating mechanism (1) is provided with a cylinder (20) with at least one, preferably two, three, four or five overflow passages (21a, 21b, 21c, 21d) for a hydraulic fluid, which are connected to the interior of the cylinder by at least one bore (22a, 22b, 22b', 22c, 22c', 22d, 22d'), and wherein a piston (30) movable along the longitudinal axis of the cylinder (20) is provided within the cylinder (20), said piston (30) partitioning the cylinder interior (23) into a first cylinder space (23a) and a second cylinder space (23b) and connecting via a piston rod (31) to the end effector (100), and that at least one fitting (41) is provided for feeding hydraulic fluid into the first cylinder space (23a) and at least one further fitting (41a, 41b, 41c, 41d) is provided, which is connected to at least one overflow passage (21a, 21b, 21c, 21d) of the cylinder (20).

2. Medical instrument according to claim 1, **characterised in that** the at least one bore (22a, 22b, 22b', 22c, 22c', 22d, 22d') of at least one overflow passage (21a, 21b, 21c, 21d) is located between an upper and a lower piston stop, in such a way that the piston (30) may be brought to at least one intermediate position in the cylinder (20), which is situated between the upper and the lower piston stop.

3. Medical instrument according to claim 1 or 2, **characterised in that** the at least one overflow passage (21a, 21b, 21c, 21d) is formed as a groove on the exterior wall of the cylinder (20) and is connected via the at least one bore (22a, 22b, 22b', 22c, 22c', 22d, 22d') to the cylinder space (23a, 23b) and that the cylinder (20) is to be inserted into a sleeve (10).

4. Medical instrument according to claim 3, **characterised in that** the fittings (41, 41a, 41b, 41c, 41d) for the hydraulic fluid, which are connected to the interior space (23) of the cylinder (20), are located in a sealing element (40) placed at the end of the sleeve (10) facing away from the piston rod (31).

5. Medical instrument according to claim 1 or 2, **characterised in that** the at least one overflow passage (21a, 21b, 21c, 21d) is located within the cylinder wall and is connected to the cylinder space (23a, 23b) by at least one bore (22a, 22b, 22b', 22c, 22c', 22d, 22d').

6. Medical instrument according to any of claims 1 to 5, **characterised in that** for each overflow passage (21a, 21b, 21c, 21d) two bores (22a, 22b, 22b', 22c, 22c', 22d, 22d') are provided.

7. Medical instrument according to any of claims 1 to 6, **characterised in that** the end effector (100) has two claw-like gripping elements (110a, 110b), which may be moved towards each other from an opened position to at least one, and preferably two, closed positions.

8. Medical instrument according to any of claims 1 to 7, **characterised in that** a device having a hydraulic system (200) for actuating an end effector of said medical instrument is provided, which cooperates with the hydraulic actuating mechanism (1) of the medical instrument, said hydraulic system (200) comprising at least one pump (201) and at least one hydraulic line (202, 202a, 202b, 202c, 202d) leading to each fitting (41, 41a, 41b, 41c, 41d) of the sealing element (40), each hydraulic line (202, 202a, 202b, 202c, 202d) preferably being provided with at least one switching element (204).

## Revendications

1. Instrument médical comportant un élément longitudinal ainsi qu'un effecteur terminal (100) pouvant être monté à une extrémité de l'élément longitudinal, cet effecteur terminal (100) pouvant être actionné par un mécanisme d'actionnement hydraulique (1),
**caractérisé en ce que**
le mécanisme d'actionnement hydraulique (1) comporte un cylindre (20) équipé d'au moins un et de préférence de deux, trois, quatre ou cinq canaux de passage (21a, 21b, 21c, 21d) d'un fluide hydraulique qui sont reliés à la partie interne du cylindre par au moins un perçage (22a, 22b, 22b', 22c, 22c', 22d, 22d'), un piston (30) monté dans le cylindre (20) et mobile le long de l'axe longitudinal du cylindre (20) est prévu, ce piston (30) subdivisant la partie interne (23) du cylindre (20) en une première chambre de cylindre (23a) et une seconde chambre de cylindre (23b), et étant relié à l'effecteur terminal (100), par l'intermédiaire d'une tige de piston (31) et, il est prévu au moins un élément de liaison (41) pour permettre l'introduction de fluide hydraulique dans la première chambre de cylindre (23a) ainsi qu'au moins un autre élément de liaison (41a, 41 b, 41c, 41d) qui est relié à au moins un canal de passage (21a, 21 b, 21c, 21d) du cylindre (20).

2. Instrument médical conforme à la revendication 1,
**caractérisé en ce que**
le perçage (22a, 22b, 22b', 22c, 22c', 22d, 22d') d'au moins un canal de passage (21a, 21 b, 21c, 2 1 d) est monté entre une butée de piston supérieure et une butée de piston inférieure de sorte que le piston (30) puisse être positionné dans le cylindre (20) dans au moins une position médiane définie qui est située entre la butée de piston supérieure et la butée de piston inférieure.

3. Instrument médical conforme à la revendication 1 ou 2,
**caractérisé en ce que**
le canal de passage (21a, 21 b, 21c, 21d) est réalisé sur la surface externe du cylindre (20) sous la forme d'un évidement et est relié à la chambre de cylindre (23a, 23b), par l'intermédiaire du perçage (22a, 22b, 22b', 22c, 22c', 22d, 22d') le cylindre (20) pouvant être logé dans une douille (10).

4. Instrument médical conforme à la revendication 3,
**caractérisé en ce que**
les éléments de liaison (41, 41a, 41b, 41c, 41d) pour le fluide hydraulique reliés à la partie interne (23) du cylindre (20) sont montés dans un élément d'étanchéité (40) qui est monté à l'extrémité de la douille (10) située à l'opposé de la tige de piston (31).

5. Instrument médical conforme à la revendication 1 ou 2,
**caractérisé en ce que**
le canal de passage (21a, 21 b, 21c, 21d) est monté dans la paroi du cylindre et est relié à la partie interne du cylindre (23a, 23b) pour l'intermédiaire d'au moins un perçage (22a, 22b, 22b', 22c, 22c', 22d, 22d').

6. Instrument médical conforme à l'une des revendications 1 à 5,
**caractérisé en ce que**
pour chaque canal de passage (21a, 21 b, 21c, 21d) il est prévu deux perçages (22a, 22b, 22b', 22c, 22c', 22d, 22d').

7. Instrument médical conforme à l'une des revendications 1 à 6,
**caractérisé en ce que**
l'effecteur terminal (100) comporte deux éléments de préhension en forme de griffes (100a, 110b) qui peuvent être déplacés à partir d'une position ouverte dans au moins une position fermée et de préférence dans deux positions fermées.

8. Instrument médical conforme à l'une des revendications 1 à 7,
**caractérisé en ce qu'**
il est prévu un dispositif d'actionnement de l'effecteur terminal de cet instrument médical avec un dispositif hydraulique (200) qui coopère avec le mécanisme d'actionnement hydraulique (1) de l'instrument médical, le dispositif hydraulique (200) comportant au moins une pompe (201) aussi qu'au moins une conduite hydraulique respective (202, 202a, 202b, 202c, 202d) s'étendant jusqu'à chaque élément de liaison (41, 41a, 41b, 41c, 41d) de l'élément d'étanchéité (40), et de préférence à chaque conduite (202, 202a, 202b, 202c, 202d) est associé au moins un élément de commutation (204).
